# EUROPÄISCHE PATENTSCHRIFT

(11) **EP 2 732 798 B1**
(45) Veröffentlichungstag und Bekanntmachung des Hinweises auf die Patenterteilung: **11.01.2017**
(21) Anmeldenummer: 13193071.1
(22) Anmeldetag: 15.11.2013
(51) Int. Cl.: A61F 2/91

(54) **Temporärer Stent**
Temporary stent
Stent temporaire

(30) Priorität: 16.11.2012 DE 102012022400
(43) Veröffentlichungstag der Anmeldung: 21.05.2014
(73) Patentinhaber: Bentley InnoMed GmbH, 72379 Hechingen (DE)
(72) Erfinder: Obradovic, Milisav, 79539 Lörrach (DE); Sunnanvädar, Lars, 72379 Hechingen (DE)
(74) Vertreter: Thiel, Christian

(56) Entgegenhaltungen:
- EP-A1- 1 997 458
- WO-A1-2006/065665
- WO-A1-2010/083770
- WO-A2-03/047460
- WO-A2-2007/058842
- US-A1- 2006 155 321
- US-A1- 2006 287 701

## Beschreibung

Die Erfindung betrifft einen temporären Stent zur Applikation von Arzneimitteln auf eine Gefäßwand. Der Stent weist im expandierten Zustand eine Maschenstruktur auf, bei der die einzelnen Maschen durch Stege begrenzt sind und Stege in Knotenpunkten zusammenlaufen.

Der Einsatz von Stents zur Beseitigung von Gefäßverengungen wird weithin praktiziert und gehört zum medizinischen Alltag. Stents, auch als Gefäßstützen bezeichnet, sollen geschwächte Gefäßwände abstützen oder durch Plaque-Ablagerungen verstopfte Gefäße wieder erweitern.

Unterschieden wird zwischen Stents, die mit Hilfe eines Ballonkatheters hydraulisch aufgeweitet werden und gegen die Gefäßwand gepresst werden und selbstexpandieren Stents, die am Ort der Implantation sich automatisch an die Gefäßwand anpassen. Verengte Gefäße werden in der Regel über Ballone oder über Ballon expandierbare Stents erweitert. Selbstexpandierende Stents werden dagegen häufig im neurologischen Sektor eingesetzt, beispielsweise auch zur Überbrückung von Gefäßfehlbildungen, wie Aneurysmen.

Die Aufweitung von Gefäßen, sei es durch Ballon expandierbare Stents oder durch Ballone, erfolgt zumeist unter einem erheblichen Druck, der wiederum zu Verletzungen der Gefäßwand führen kann. In der Folge kommt es, auch nach der Platzierung eines Stents, zu sogenannten Restenosen, d. h. die Reizung oder Verletzung der Gefäßwand durch die Platzierung des Stents oder die Ausübung von Druck über den Ballon stimuliert das Wachstum von körpereigenem Gewebe in das Gefäß und zu erneuter Verengung. Es handelt sich dabei um einen kurzfristig ablaufenden Prozess, der mit der Abheilung der Gefäßwand oder Beendigung des Reizes zum Erliegen kommt. Die Komplikationen können allerdings erheblich sein.

Zur Verhinderung solcher Restenosen wurden medikamentenbeschichtete Stents entwickelt, die über einen gewissen Zeitraum Wirkstoff abgeben, die die Zellproliferation hemmen. Das Konzept hat sich als wirksam erwiesen. Die Wirkstoffabgabe ist nur über einen gewissen Zeitraum, etwa zwei Wochen, erforderlich; anschließend ist die Gefahr einer Restenose deutlich verringert. Allerdings kann auch dann der implantierte Stent noch über bewegungsbedingte Reizung oder Unverträglichkeit zu Restenoseerscheinungen führen. Im Grunde genommen kann nach Beseitigung des Engpasses und Abheilung etwaiger Verletzungen der Gefäßwand auf den Stent verzichtet werden.

Das Prinzip der wirkstoffbeschichteten Stents wurde vielfach variiert und kommt in zahlreichen Varianten zum Einsatz. Zumeist ist der Stent zumindest auf seiner Außenseite mit einer Beschichtung versehen, die in einer polymeren oder sonstigen Matrix den Wirkstoff enthält. Übliche Wirkstoffe sind Paclitaxel und Rapamycin, beides proliferationshemmende Substanzen. Der Wirkstoff wird über eine vorbestimmte Zeit abgegeben, anschließend ist das Reservoir erschöpft. Soweit eine Stützung der Gefäßwand nicht mehr erforderlich ist, ist der Stent funktionslos.

Beschichtete Stents haben darüber hinaus den Nachteil, dass die auf der Außenseite aufgebrachte medikamentenhaltige Beschichtung bei der Platzierung beschädigt oder teilweise abgetragen wird. Der Expansionsprozess verursacht zudem Risse und Spannungen, die das Abgabeverhalten für den Wirkstoff beeinflussen. Darunter leidet die Zuverlässigkeit der Wirkstoffabgabe.

Die EP 1 997 458 A1 offenbart einen rückziehbaren Stent zur Applikation von Arzneimitteln, dessen Grundstruktur aus verformbaren Streben stoffschlüssig zusammengesetzt ist und durch eine an diese Streben angebundene Feinstruktur aus einem draht- oder fadenartigen Material ergänzt wird. Nachteilig ist neben der aufwändigen und entsprechend kostspieligen Herstellung eines solchen Implantates auch, dass die Materialien sich voneinander lösen und zu Komplikationen gerade bei der angestrebten komplikationslosen Entfernung des Stents führen können.

Aufgabe der Erfindung ist es zum Einen, ein verlässliches System für die Wirkstoffabgabe zu schaffen, das diesem Nachteil nicht unterliegt und zum anderen einen Stent bereitzustellen, der nach der Wirkstoffabgabe und Erfüllung seiner Funktion ohne die genannten Nachteile bekannter Systeme wieder entfernt werden kann.

Diese Aufgabe wird mit einem Stent der eingangs beschriebenen Art gelöst, der an seinem proximalen Ende wenigstens ein Mittel zur Festlegung eines Extraktionsgeräts aufweist und bei dem wenigstens ein Teil der Stege und/oder Knotenpunkte an der Außenseite mit Eintiefungen versehen ist, in denen ein Arzneimittel enthalten ist.

Der erfindungsgemäße Stent ist ein Stent mit einer Maschen- oder Wabenstruktur, bei der die einzelnen Maschen bzw. Waben durch Stege begrenzt sind. Die einzelnen Maschen haben eine gestaffelte Anordnung, d. h. die einzelnen Maschen sind nach Art eines Maschendrahtzaunes angeordnet. Die einzelnen Maschen sind durch Stege begrenzt, wobei aneinandergrenzende Maschen gemeinsame Stege aufweisen. Die Stege der Maschen laufen in einzelnen Knotenpunkten zusammen, in der Regel drei oder vier Stege aneinandergrenzender Maschen in einem Knotenpunkt.

Der erfindungsgemäße Stent weist an seinem proximalen Ende, d. h. dem Ende, das dem Einführungskatheter zugewandt ist, wenigstens ein Mittel zur Festlegung eines Extraktionsgerätes auf. Es kann sich dabei vorzugsweise um einen oder mehrere Haken handeln. An seiner Außenseite, d. h. der gefäßzugewandten Seite, weist der Stent Eintiefungen in den Stegen und/oder den Knotenpunkten auf, in der das jeweils interessierende Arzneimittel enthalten ist. Als Arzneimittel kommen insbesondere proliferationshemmende Mittel, etwa Rapamycin oder Paclitaxel, aber auch entzündungshemmende Mittel, Antibiotika oder dergleichen in Frage. Derartige Mittel sind dem Fachmann vertraut. In der Regel befinden sich die Arzneimittel in einer Polymermatrix, welche wiederum die Stege stabiler macht.

Der erfindungsgemäße Stent ist vorzugsweise selbstexpandierend und besteht aus einem Formgedächtnismaterial. Dies kann ein Federstahl sein, ist insbesondere aber eine Nickel-Titan-Legierung, etwa Nitinol. Derartige selbstexpandierende Stents werden vorzugsweise aus einem Rohr des entsprechenden Materials mit Hilfe eines Lasers geschnitten, in die expandierte Form gebracht und in dieser fixiert. Für die Platzierung wird der Stent dann in kontrahierter Form in einen Katheter gebracht, an den Einsatzort buchsiert und dort freigesetzt. Nach Ausbringung aus dem Katheter nimmt er die expandierte Form an und positioniert sich selbsttätig gegen die zu behandelnde Gefäßwand.

Es versteht sich, dass die Erfindung auch ballonexpandierbare Stents erfasst.

Das wenigstens eine Mittel zur Festlegung eines Extraktionsgeräts ist zweckmäßigerweise zentrisch zur Stentachse angeordnet. Dies bedeutet, dass dieses Festlegungsmittel zu allen Seiten zur Gefäßwand beabstandet und für ein Extraktionsgerät, beispielsweise einen Retriever in Form eines Führungsdrahts mit Haken, zugänglich ist.

Zweckmäßigerweise weist der erfindungsgemäße Stent an seinem proximalen Ende einen oder mehrere Haken auf.

Haken und Ösen werden aus den Stegen geformt, die das proximale Ende des erfindungsgemäßen Stents ausmachen. Die um die Zirkumferenz herum angeordneten Waben laufen am proximalen Ende in einzelnen Stegen aus, die proximal zu einer oder mehreren Ösen verbunden sein können. In diese Ösen kann beispielsweise der Haken eines Retrievers einhaken. Es ist ferner möglich, die miteinander verbundenen Stegenden zu einem Haken, der zweckmäßigerweise zur Gefäßmitte hin ausgebildet ist, umzubiegen. In so einem Haken findet ein Retriever mehrere Ansatzpunkte für die Festlegung und Extraktion.

Für die Extraktion wird der Stent dann mit dem Retriever in einen Katheter gezogen, wobei er sich automatisch wieder zur Rohrform zusammenzieht, und aus dem Körper ausgebracht wird. Die Wabenstruktur ist geeignet, den Kontraktionsprozess zu fördern, ebenso die Eintiefungen bzw. Einschnitte in die Stege, die nach Abgabe ihrer Arzneimittelfüllung an Stabilität verlieren. Dadurch vermindert sich die Radialkraft des Stents, was das Einziehen in einen Katheter erleichtert.

Die Eintiefungen in den Stegen und/oder Knotenpunkten des erfindungsgemäßen Stents befinden sich auf der Außenseite und insbesondere im zentralen Stentbereich. Der zentrale Stentbereich ist der Bereich, der sich eng an die Gefäßwand anlegt und geeignet ist, das in die eingetieften Bereiche eingebrachte Medikament auf die Gefäßwand zu übertragen.

In der Regel sind nur die Stege des zentralen Stentbereichs mit solchen Eintiefungen versehen. Die Eintiefungen sind beispielsweise Einschnitte in Rinnenform, die mit dem Medikament oder einer Medikamentenzubereitung gefüllt sind. In diesen Eintiefungen sind die darin enthaltenen Wirkstoffe vor der Abtragung beispielsweise durch eine Katheterwand geschützt. Da sich die Form der Stege bei der Selbstexpansion nicht verändert - es ändert sich allein die Maschenstruktur - kommt die Medikamentenfüllung der Eintiefungen in unveränderter Form und unbeeinflusst von Expansionsprozessen zur Anwendung.

Zweckmäßigerweise bleiben die Knotenpunkte frei von Eintiefungen. Dies hat den Vorteil, dass die Knotenpunkte, die ja bei der Expansion und Umformung des Stents die Spannungen aufnehmen müssen, nicht geschwächt werden. Ansonsten bestünde die Gefahr, dass der Stent bei der Umformung in seine expandierte Form im Bereich der Knotenpunkte Schaden nimmt, beispielsweise dass Stege abreißen.

Aus diesem Grund ist es auch zweckmäßig, die Knotenpunkte so auszubilden, dass sich die darin zusammengeführten Stege im Verlauf kreuzen. Dies bedeutet, dass die in der Längsrichtung ausgerichteten Maschen an ihren Spitzen gegeneinander etwas versetzt sind; die bei der Ausbildung der Maschenstruktur gesetzten Laserschnitte laufen dann nicht aufeinander zu und führen nicht zum Reißen der Struktur bei der Expansion. Aus dem gleichen Grunde enden die Eintiefungen in den Stegen zweckmäßigerweise vor den Knotenpunkten; die an die Knotenpunkte angrenzenden Bereiche der Stege verbleiben ohne Eintiefungen.

Die erfindungsgemäßen Stents sind dafür bestimmt, nach einer kurzen Anwendung, häufig schon nach 6 Tagen, wieder explantiert zu werden. In der Zwischenzeit haben sie ihren Medikamentenvorrat, der in den Eintiefungen gespeichert ist, an die Gefäßwand abgegeben.

Die Platzierung des Stents im Gefäßsystems erfolgt auf herkömmliche Art und Weise. Bei selbstexpandierenden Stents wird der Stent mit Hilfe eines Katheters an den Einsatzort gebracht und dort aus dem Katheter hinausgeschoben. Bei ballonexpandierbaren Stents wird der Stent vor dem Einsatz auf einen Ballon gekrimpt, mit einem Katheter an den Einsatzort gebracht und dort aus dem Katheter freigesetzt und über den expandierten Ballon an die Gefäßwand gedrückt. Der druckentlastete Ballon wird anschließend mit dem Katheter zurückgezogen.

Die Rückholung des Stents erfolgt über die Mittel zur Festlegung des Extraktionsgerätes, etwa eines Führungsdrahts mit einer Schlinge oder einem Haken am distalen Ende. Der Stent wird über den Haken oder die Öse am Rückholmittel festgelegt, in einen Katheter gezogen, wobei er sich wieder zur Rohrform verschlankt und aus dem Gefäßsystem herausgezogen wird.

Die Erfindung wird durch die beiliegenden Abbildungen näher erläutert. Es zeigen:
- Figur 1: einen nicht erfindungsgemäßen Stent in der Gesamtansicht;
- Figur 2: das nicht erfindungsgemäße proximale Ende des Stents gemäß Abbildung 1;
- Figur 3: ein Detail aus dem zentralen Abschnitt eines erfindungsgemäßen Stents und
- Figur 4: das proximale Ende eines erfindungsgemäßen Stents.

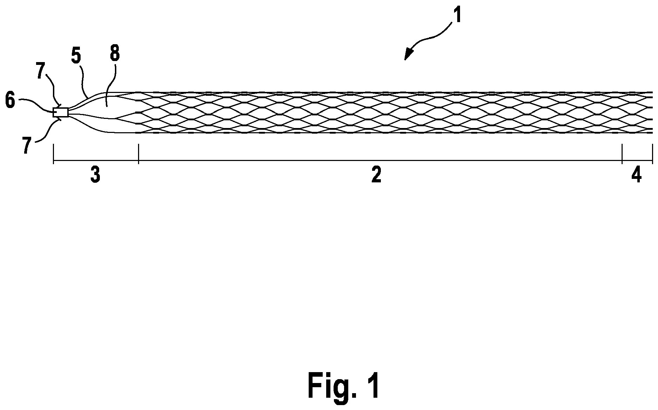

Figur 1 zeigt einen erfindungsgemäßen Stent in der Gesamtansicht mit einem nicht erfindungsgemäßen proximalen Ende 3, einem zentralen Bereich 2 und einem distalen Ende 4. Das distale Ende 4 endet in auslaufenden Maschen, die zur besseren Platzierung mit röntgensichtbaren Markerelementen versehen sein können (nicht dargestellt). Der zentrale Bereich 2 ist der Bereich, in dem sich die Stege und/oder Knotenpunkte mit den Eintiefungen befinden, die das Arzneimittel enthalten. Das proximale Ende 3 läuft in einzelne Stege 5 aus, die proximal nicht erfindungsgemäß in einen rohrförmigen Abschnitt 6 zusammengefasst sind.

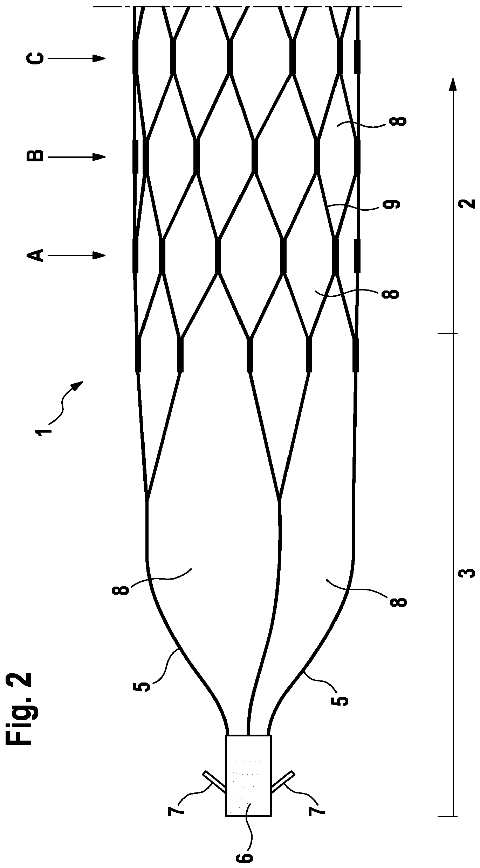

Figur 2 zeigt eine vergrößerte Ansicht des nicht erfindungsgemäßen proximalen Endes des Stents gemäß Abbildung 1 mit dem rohrförmigen Abschnitt 6 und den beiden ausgestellten Haken 7.

Die dargestellte, voll expandierte Form resultiert aus einer mit an und für sich bekannten Mitteln erfolgten Aufweitung und Fixierung der expandierten Form. Bevorzugtes Material für die erfindungsgemäßen Stents ist bei ballonexpandierbaren Modellen ein üblicher medizinischer Stahl, bei selbstexpandierenden Modellen ein Federstahl oder eine Formgedächtnislegierung, etwa Nitinol.

Deutlich zu erkennen ist, dass im sich distal anschließenden zentralen Bereich des Stents die Maschen oder Waben 8 deutlich enger sind und ein regelmäßiges Muster von sich aneinanderreihenden Ringsegmenten A, B und C ausbilden. Die einzelnen Ringsegmente sind gestaffelt angeordnet, so dass sich ein Maschenmuster nach Art eines Maschendrahtzaunes ergibt. Die Ringsegmente A, B und C sind jeweils über gemeinsame Stege 9 miteinander verbunden.

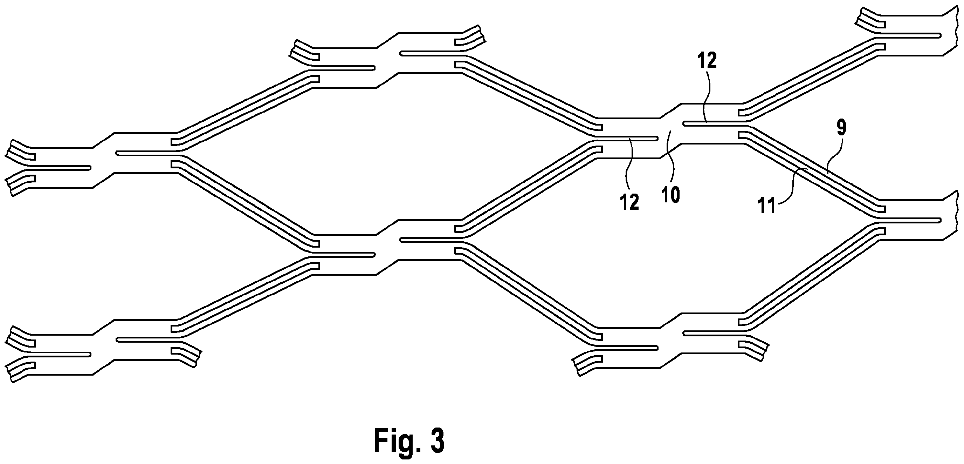

Figur 3 zeigt einen zentralen Stentabschnitt mit den einzelnen Stegen 9 und den Knotenpunkten 10, in dem jeweils vier Stege aneinandergrenzender Maschen 8 zusammenlaufen. Die Stege 9 haben in ihrem Verlauf eine Eintiefung 11, die rinnenförmig ausgebildet ist. Diese Eintiefungen werden beim Schneiden des Stents aus dem Rohr mit Hilfe des Lasers erzeugt; der Laser (Femtolaser) ist so eingestellt, dass er zwar Material abträgt bzw. verdampft, jedoch den Steg nur aushöhlt, nicht aufspaltet. Die Eintiefungen 11 sind im erfindungsgemäßen Stent mit der Arzneimittelformulierung gefüllt, beispielsweise dem Wirkstoff in einer polymeren Trägermatrix, die den Wirkstoff über den gewünschten Zeitraum abgibt. Als Trägermatrix haben sich die für die Beschichtung von Stents verwandten polymeren Materialien bewährt, die außerdem mit ihrer Füllung dem Steg zusätzliche Stabilität verleihen. Bevorzugt sind bioresorbierbare Polymere vom Typ der Resomere^{®}.

Die die Maschen begrenzenden Stege 9 laufen in Knotenpunkten 10 zusammen. Dabei ist die Verbindung in den Knotenpunkten 10so gestaltet, dass ein geringer Versatz da ist, der es ermöglicht, dass sich ein Kreuzungsmuster ergibt. Beim Schneiden des Stents wird so verfahren, dass die einzelnen Maschen um ein Geringes gegeneinander versetzt sind, so dass die Einschnitte 12 in die Knotenpunkte 10 nicht aufeinander zulaufen. Dies verhindert, dass der Stent beim Expandieren an dieser Stelle reißt. Der Stabilität dient auch, dass die Knotenpunkte 10 keine Taschen oder sonstigen Eintiefungen enthalten und damit ihre volle Stabilität bewahren.

Es versteht sich, dass im zentralen Bereich des Stents die Knotenpunkte 10 jeweils von vier Stegen 9 gebildet werden, dagegen im proximalen Bereich 3 mit den erweiterten Maschen nur von drei Stegen 5 bzw. 9.

Zu sehen ist, dass die rinnenförmigen Eintiefungen 11 in die Stege 9 nicht über die gesamte Steglänge gehen, sondern vor den Knotenpunkten 10 enden, um auf diese Weise die Stabilität des Stents zu erhöhen. Stabile Knotenpunkte bedeuten auch, dass der Stent eine hinreichende Radialkraft hat und die Stege 9 mittels der Knotenpunkte 10 an der Gefäßwand gehalten werden.

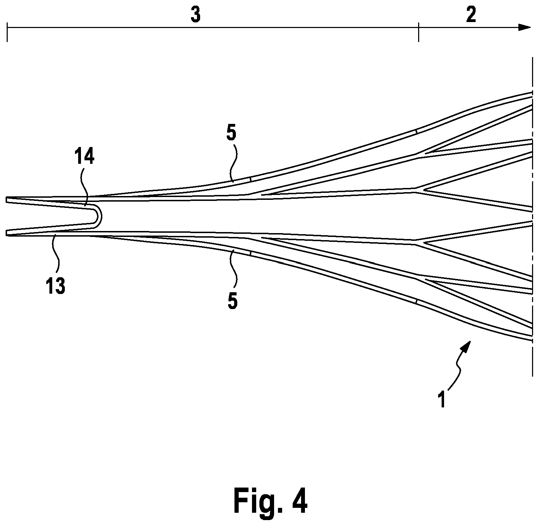

Figur 4 zeigt eine erfindungsgemäße Ausführungsform des proximalen Abschnitts 3 eines erfindungsgemäßen Stents. Im proximalen Bereich 3 sind die Stege 5, im dargestellten Fall vier Stege 5 so zusammengeführt, dass sie eine Schlaufe 13 bilden, deren proximales Ende zu einem Haken 14 nach distal zurückgebogen ist. Alternativ kann das proximale Ende auch als reine Schlaufe oder Öse ausgebildet sein. Auch hier ist das proximale Ende so ausgestaltet, dass der Haken 14 oder die entsprechende Schlaufe bzw. Öse in etwa entlang der Stentachse orientiert ist, also in der Mitte des Blutgefäßes liegt und über einen Retriever einfach zu greifen ist.

## Patentansprüche

1. Temporärer Stent, insbesondere zur Applikation von Arzneimitteln auf eine Gefäßwand, mit einer Maschenstruktur, bei der die einzelnen Maschen (8) durch Stege (9, 5) begrenzt sind, wobei die Stege in Knotenpunkten (10) zusammenlaufen und wenigstens einen Teil der Stege (9) und/oder Knotenpunkte (10) an der Außenseite Eintiefungen (11) aufweist, in denen Arzneimittel enthalten sind, und mit Mitteln zur Festlegung eines Extraktionsgerätes am proximalen Ende, **dadurch gekennzeichnet, dass** die Knotenpunkte (10) von einander kreuzenden Stegen (9) gebildet werden, dergestalt, dass die Spitzen von im Längsverlauf des Stents (1) aufeinanderfolgende Maschen (8) gegeneinander versetzt sind; wobei die Mittel zur Festlegung des Extraktionsgerätes zentrisch zur Stentachse angeordnet und als Haken ausgebildet sind, wobei wenigstens zwei proximale Stegenden (13) so zusammengeführt sind, dass sie eine Schlaufe (14) bilden, deren proximales Ende nach distal gebogen den Haken bildet.

2. Stent nach Anspruch 1, **dadurch gekennzeichnet, dass** er selbstexpandierend ist.

3. Stent nach Anspruch 1 oder 2, **dadurch gekennzeichnet, dass** er aus einem Formgedächtnismaterial besteht, insbesondere aus einer Nickel-Titan-Legierung.

4. Stent nach Anspruch 1, **dadurch gekennzeichnet, dass** er ballonexpandierbar ist.

5. Stent nach einem der vorstehenden Ansprüche, **dadurch gekennzeichnet, dass** er aus einem Rohr geschnitten und in seine expandierte Form umgeformt ist.

6. Stent nach einem der vorstehenden Ansprüche, **dadurch gekennzeichnet, dass** die Stege (9) des zentralen (2) Stentbereichs Arzneimittel enthaltende Eintiefungen (11) aufweisen.

7. Stent nach Anspruch 6, **dadurch gekennzeichnet, dass** die Eintiefungen (11) Rinnenform haben.

8. Stent nach einem der vorstehenden Ansprüche, **dadurch gekennzeichnet, dass** die Stege (8) des zentralen Bereichs (2) an den an die Knotenpunkte angrenzenden Enden ohne Eintiefungen (11) bleiben.

## Claims

1. Temporary stent, in particular for applying medicaments to a vascular wall, having a mesh structure in which the individual loops (8) are delimited by bridges (9, 5), wherein the bridges converge in intersection points (10) and at least part of the bridges (9) and/or of the intersection points (10) on the external side have depressions (11) in which medicaments are contained, and having means for fixing an extraction apparatus at the proximal end, **characterized in that** the intersection points (10) are formed by mutually crossing bridges (9) in such a manner that the tips of loops (8) which are sequential in the longitudinal profile of the stent (1) are mutually offset, wherein the means for fixing the extraction apparatus are disposed so as to be centrical to the stent axis and are configured as hooks, wherein at least two proximal bridge ends (13) are gathered such that the latter form a noose (14) of which the proximal end, bent in the distal direction, forms the hook.

2. Stent according to Claim 1, **characterized in that** said stent is self-expanding.

3. Stent according to Claim 1 or 2, **characterized in that** said stent is composed of shape-memory material, in particular of a nickel-titanium alloy.

4. Stent according to Claim 1, **characterized in that** said stent is expandable in the manner of a balloon.

5. Stent according to one of the preceding claims, **characterized in that** said stent is cut from a tube and said stent is formed into its expanded shape.

6. Stent according to one of the preceding claims, **characterized in that** the bridges (9) of the central stent region (2) have medicament-containing depressions (11).

7. Stent according to Claim 6, **characterized in that** the depressions (11) have the shape of trenches.

8. Stent according to one of the preceding claims, **characterized in that** the bridges (8) of the central region (2) at the ends thereof that are adjacent to the intersection points remain without depressions (11).

## Revendications

1. Stent temporaire, en particulier pour l'application de médicaments sur une paroi de vaisseau, avec une structure maillée pour laquelle les mailles individuelles (8) sont délimitées par des nervures (9, 5), dans lequel les nervures convergent au niveau de points de croisement (10) et au moins une partie des nervures (9) et/ou points de croisement (10) présentent des creux (11) sur le côté extérieur, lesquels contiennent des médicaments, et avec des moyens pour la fixation d'un appareil d'extraction à l'extrémité proximale,
**caractérisé en ce que**
les points de croisement (10) sont formés par des nervures (9) se croisant les unes les autres de manière à ce que les pointes de mailles (8) successives sur le tracé longitudinal du stent (1) soient décalées les unes par rapport aux autres ; dans lequel les moyens pour la fixation de l'appareil d'extraction sont disposés centralement par rapport à l'axe de stent et sont réalisés en tant que crochets, dans lequel au moins deux extrémités de nervure proximales (13) sont réunies d'une manière telle, qu'elles forment une boucle (14) dont l'extrémité proximale, courbée dans la

2. Stent selon la revendication 1, **caractérisé en ce qu'**il est auto-expansible.

3. Stent selon la revendication 1 ou 2, **caractérisé en ce qu'**il se compose d'un matériau à mémoire de forme, en particulier d'un alliage de nickel-titane.

4. Stent selon la revendication 1, **caractérisé en ce qu'**il est expansible par ballonnet.

5. Stent selon l'une des revendications précédentes, **caractérisé en ce qu'**il est découpé à partir d'un tuyau et que sa forme est modifiée en sa forme expansée.

6. Stent selon l'une des revendications précédentes, **caractérisé en ce que** les nervures (9) de la zone de stent centrale (2) présentent des creux (11) contenant des médicaments.

7. Stent selon la revendication 6, **caractérisé en ce que** les creux (11) ont une forme de rigole.

8. Stent selon l'une des revendications précédentes, **caractérisé en ce que** les nervures (8) de la zone centrale (2) restent sans creux (11) aux extrémités jouxtant les points de croisement.
